# EUROPÄISCHE PATENTANMELDUNG

(11) **EP 3 569 276 A1**
(43) Veröffentlichungstag der Anmeldung: **20.11.2019**
(21) Anmeldenummer: 18172884.1
(22) Anmeldetag: 17.05.2018
(51) Int. Cl.: A61M 16/00, A61M 16/20, A61M 15/00, A61M 16/06, A61M 11/00, A61M 16/14, A61M 11/06, A61M 11/02

(54) **INHALIERVORRICHTUNGSKOMPONENTE UND VIBRATIONSSYSTEM**

(71) Anmelder: PARI GmbH Spezialisten für effektive Inhalation, 82319 Starnberg (DE)
(72) Erfinder: Neemann, Carsten, 81241 München (DE); Schwenck, Nicolas, 81375 München (DE)
(74) Vertreter: Hoffmann Eitle

(57) **Zusammenfassung**

Die Erfindung betrifft eine Inhaliervorrichtungskomponente (30) mit
einem Auslassanschluss (2), der dazu eingerichtet ist, mit einem Auslass (8) einer Verdrängermaschine (9) durchströmbar verbunden zu werden, und
einem Vibrationseinlassanschluss (6), der dazu eingerichtet ist, mit einer Vibrationsschnittstelle (13) durchströmbar verbunden zu werden,
einem Vibrationsventil (1), das
eine Vibrations-Schaltstellung, in der der Auslassanschluss (2) mit dem Vibrationseinlassanschluss (6) durchströmbar verbunden ist, und
eine Aerosolerzeugungs-Schaltstellung, in der die Verbindung zwischen Auslassanschluss (2) und Vibrationseinlassanschluss (6) gesperrt ist, aufweist,
und einer Schalteinrichtung (19) zum Schalten des Vibrationsventils (1).

## Beschreibung

Die Erfindung betrifft eine Inhaliervorrichtungskomponente, eine Vibrationsinhaliervorrichtung, ein Vibrationssystem und die Verwendung einer Inhaliervorrichtungskomponente.

Im Stand der Technik sind Inhaliervorrichtungen bekannt, die sowohl ein Aerosol als auch eine Vibration bereitstellen, um eine Aerosoldeposition in einem schwer zugänglichen Bereich zu erreichen. Eine derartige Inhaliervorrichtung ist in EP1534370B1 beschrieben.

Aufgabe der vorliegenden Erfindung ist es, eine Möglichkeit bereitzustellen, mit der Aerosol und Vibration auf einfache Weise bereitgestellt werden können. Diese Aufgabe wird gelöst durch eine Inhaliervorrichtungskomponente mit einem Auslassanschluss, der dazu eingerichtet ist, mit einem Auslass einer Verdrängermaschine durchströmbar verbunden zu werden, und einem Vibrationseinlassanschluss, der dazu eingerichtet ist, mit einer Vibrationsschnittstelle durchströmbar verbunden zu werden, einem Vibrationsventil, das eine Vibrations-Schaltstellung, in der der Auslassanschluss mit dem Vibrationseinlassanschluss durchströmbar verbunden ist, und eine Aerosolerzeugungs-Schaltstellung, in der die Verbindung zwischen Auslassanschluss und Vibrationseinlassanschluss gesperrt ist, aufweist, und einer Schalteinrichtung zum Schalten des Vibrationsventils.

In einer Ausführungsform ist die Inhaliervorrichtungskomponente dazu eingerichtet, eine bestehende Inhaliervorrichtung, bevorzugt ein Verdrängermaschine-Inhaliervorrichtungssystem, besonders bevorzugt ein Kompressor-Inhaliervorrichtungssystem oder ein Kompressor-Düsenverneblersystem zu einer Inhaliervorrichtung zur Nebenhöhlendeposition oder zur Deposition in Bronchiektasen zu erweitern. In einer Ausführungsform ist die Inhaliervorrichtungskomponente als Zusatzmodul ausgebildet, welches ein Anwender oder Patient an einen vorhandenen Kompressor anschließen kann. Bevorzugt ist die Inhaliervorrichtungskomponente als einfache Strömungs-Vibrations-Bereitstellungseinrichtung für einen Aerosoltransport in eine Nasennebenhöhle oder mehrere Nasennebenhöhlen ausgebildet. In einer Ausführungsform ist die Inhaliervorrichtungskomponente derart ausgebildet, dass mit einer einzigen Verdrängermaschine Aerosol gefördert und vibriert werden kann. In einer besonders bevorzugten Ausführungsform ist das Vibrationsventil derart ausgebildet, dass mit einer einzigen Verdrängermaschine Aerosol erzeugt, gefördert und vibriert werden kann.

Das Vibrationsventil weist vorzugsweise ein Wegeventil auf. Zweckmäßigerweise weist das Vibrationsventil einen Schieber auf.

Die Schalteinrichtung weist vorzugsweise eine elektrische, elektromagnetische, pneumatische, hydraulische, mechanische oder manuelle Betätigungseinrichtung oder eine Kombination daraus auf. In einer Ausführungsform weist die Schalteinrichtung einen Taster auf. Die Schalteinrichtung ist vorzugsweise dazu eingerichtet, das Vibrationsventil zu schalten, besonders bevorzugt ist die Schalteinrichtung dazu eingerichtet, das Vibrationsventil zu steuern.

Besonders bevorzugt ist das Vibrationsventil elektrisch oder mechanisch durch einen Anwender schaltbar. In einer Ausführungsform ist das Vibrationsventil atemzuggesteuert, vorzugsweise in Verbindung mit einem Mundstück, das einen Sensor aufweist. In einer bevorzugten Ausführungsform ist das Vibrationsventil dazu vorgesehen, von einem Anwender oder Patienten beim Ausatmen in die Vibrations-Schaltstellung geschaltet zu werden.

Eine Verdrängermaschine ist eine Fluidenergiemaschine, bei der ein Fluid die Maschine in einzelnen Volumina durchläuft. Zweckmäßigerweise ist die Verdrängermaschine eingerichtet, eine Fluidströmung, die Volumenstromschwankungen aufweist, zu erzeugen.

Die Verdrängermaschine weist vorzugsweise einen Verdichter, einen Kompressor, eine Pumpe, eine Kolbenmaschine, eine Hubkolbenmaschine, einen Hubkolbenverdichter, eine Hubkolbenpumpe, eine Rotationskolbenmaschine, einen Kolbenverdichter, eine Kolbenpumpe, eine Schraubenmaschine, eine Membranpumpe, einen Membranverdichter, eine Schlauchpumpe, einen Schlauchverdichter, eine Exzenterschneckenpumpe, einen Exzenterschneckenverdichter, eine Impellerpumpe, einen Impellerverdichter, eine rotierende Verdrängerpumpe, einen rotierenden Verdrängerverdichter, eine Zahnradpumpe, einen Zahnradverdichter, eine Kreiskolbenpumpe, einen Kreiskolbenverdichter, eine Flügelzellenpumpe, einen Flügelzellenverdichter, einen Schraubenverdichter mit stufenweiser Veränderung der Gewindesteigungen in Abschnitten der Rotoren oder eine Kombination davon auf. Einige Ausführungsformen weisen verschiedene Kompressoren auf, die unterschiedliche Hubvolumina zur Verfügung stellen können.

Ein Fluid weist ein Gas, eine Flüssigkeit oder eine Mischung davon auf. Vorzugsweise weist das Fluid einen vibrierenden Volumenstrom auf. Eine Fluidströmung weist eine Gas- oder Flüssigkeitsströmung oder eine Mischung daraus auf.
Die Vibrationsschnittstelle ist zweckmäßigerweise eine Öffnung eines Hohlkörpers einer Inhaliervorrichtung, vorzugsweise eine Öffnung eines Inhaliervorrichtungsgehäuses. Die Vibrationsschnittstelle weist vorzugsweise einen niedrigen Strömungswiderstand auf, um durch die Verdrängermaschine erzeugte Vibrationen möglichst gut zu erhalten.

Vorzugsweise weist die Inhaliervorrichtung einen Vernebler, einen Pulverinhalator, einen Druckgas-Dosierinhalator, einen Normaldruck-Dosierinhalator, einen Zerstäuber, einen Befeuchter, einen Druckluftvernebler, einen Luftzerstäuber, einen elektronischen Vernebler, einen Ultraschallvernebler, einen elektrohydrodynamischen Vernebler, einen elektrostatischen Vernebler, einen Membranvernebler, einen Vernebler mit einer vibrierenden Membran, einen elektronischen Vernebler mit einer vibrierenden Membran, einen Mesh-Vernebler, einen Düsenvernebler, einen Inhalator (MDI), einen Pulverzerstäuber (DPI), einen Spacer, eine Chamber oder eine Kombination davon auf. Der Inhalator weist in einer Ausführungsform einen unter Druck stehenden Kanister mit einem Medikament und einem Treibgas auf. Es ist vorteilhaft, wenn der Kanister mit einem von Hand bedienbaren Aktuator verbunden ist. Zweckmäßigerweise ist der Inhalator eingerichtet, bei der Aktivierung eine bestimmte Medikamentenmenge in Aerosolform abzugeben. In einer Ausführungsform ist die Inhaliervorrichtung für den Einsatz mit Beatmungsvorrichtungen eingerichtet.

Ein Aerosol ist ein Gemisch aus festen oder flüssigen Schwebeteilchen und einem Gas.

Die Vibrations-Schaltstellung ist eine Schaltstellung des Vibrationsventils, die zweckmäßigerweise dazu vorgesehen ist, ein Aerosol in einem schwer zugänglichen Bereich zu deponieren.

Die Aerosolerzeugungs-Schaltstellung ist eine Schaltstellung des Vibrationsventils, die zweckmäßigerweise dazu vorgesehen ist, eine Aerosolerzeugung zu ermöglichen.

Der Vibrationseinlassanschluss ist gesperrt, wenn keine oder nahezu keine durchströmbare Verbindung des Vibrationseinlassanschlusses zu dem Auslassanschluss vorhanden ist.

Es ist zweckmäßig, wenn die Schaltzustände des Ventils eine Aerosolerzeugung, Vibrationserzeugung, Vibrationsleitung oder eine Kombination daraus ermöglichen. Bevorzugt sind die Zustände oder Schaltzustände bei einer Nutzung oder Anwendung schaltbar.

In einer Ausführungsform ist das Vibrationsventil zu einem Einlass einer Verdrängermaschine oder einem Lufteinlass eines Kompressors verbindungslos. Das Risiko, dass Aerosol oder Ausatemluft aus dem Vernebler in den Kompressor gesaugt wird, kann so vermieden werden. Es ist auf einfache Weise eine große Kompatibilität zwischen dem Vibrationsventil und auf dem Markt befindlichen Inhaliervorrichtungen oder Inhalations-Kompressoren erreichbar. Es ist zweckmäßig, wenn das Vibrationsventil mit vielen Inhalations-Kompressoren verbunden werden kann. Zweckmäßigerweise ist das Vibrationsventil derart ausgebildet, dass viele Kompressoren zu einem Therapiegerät für die Nasennebenhöhlen erweiterbar sind. Eine reduzierte Anzahl an Verbindungsschläuchen kann zu einer einfacheren Handhabung für den Anwender führen.

Das Vibrationsventil ist zweckmäßigerweise auf ein gefordertes Strömungsmuster und die geforderte Ansteuermöglichkeit, vorzugsweise mechanisch oder elektrisch, abgestimmt. In einer Ausführungsform ist das Vibrationsventil dazu eingerichtet, gleichzeitig eine gleichmäßige und eine vibrierende Strömung zu ermöglichen. Bevorzugt ist das Vibrationsventil dazu eingerichtet, abwechselnde Muster oder einen Wechsel zwischen gleichmäßiger und vibrierender Strömung zu ermöglichen.

In einer Ausführungsform weist das Vibrationsventil einen Fluidanschluss auf, der dazu eingerichtet ist, mit einem Fördereinlass einer Inhaliervorrichtung durchströmbar verbunden zu werden, wobei in der Aerosolerzeugungs-Schaltstellung der Auslassanschluss mit dem Fluidanschluss durchströmbar verbunden ist. So kann auf einfache Weise eine Aerosolerzeugung eingeschaltet werden.

Vorzugsweise ist der Fördereinlass eine Düse eines Düsenverneblers.

Ein Düsenvernebler weist eine Düse auf, die dazu vorgesehen ist, mit einem Gasstrom und einer Flüssigkeit ein Aerosol zu erzeugen. Vorzugsweise ist eine Kapillare dazu vorgesehen, eine Flüssigkeit zu einer Düsenöffnung zu leiten, die dazu vorgesehen ist, von dem Gas durchströmt zu werden. Zweckmäßigerweise ist die Düse ein Zweistoffsystem.

Es ist vorteilhaft, wenn das Vibrationsventil einen durchströmbaren Einlassanschluss aufweist, der dazu eingerichtet ist, mit einem Einlass der Verdrängermaschine durchströmbar verbunden zu werden, und in der Vibrations-Schaltstellung der Einlassanschluss, der Auslassanschluss oder beide mit einer Vibrationsschnittstelle durchströmbar verbindbar sind.

Auf diese Weise kann ein Fluid auf einfache Weise abwechselnd angesaugt und ausgeschoben werden.

Vorzugsweise ist der Einlass der Verdrängermaschine mit einem Ansaugventil eines Kolbenkompressors durchströmbar verbunden. In einer Ausführungsform ist eine Verbindung im Inneren des Vibrationsventils vorhanden, die in der Vibrations-Schaltstellung den Einlassanschluss mit dem Auslassanschluss durchströmbar verbindet. In einer Ausführungsform ist eine Verbindungsleitung vorhanden, die Ein- und Auslassanschluss durchströmbar verbindet, eine Umgebungsöffnung und eine Patientenöffnung aufweist, wobei ein Ventil dazu eingerichtet ist, in einer Aerosolerzeugungs-Schaltstellung den Einlassanschluss mit der Umgebungsöffnung durchströmbar zu verbinden und die Verbindung zwischen dem Einlassanschluss und dem Auslassanschluss zu sperren und in einer Vibrations-Schaltstellung die Umgebungsöffnung zu sperren und die Verbindung zwischen dem Einlassanschluss und dem Auslassanschluss freizugeben. Die Umgebungsöffnung ist zweckmäßigerweise dazu eingerichtet, Luft aus einer Umgebung in den Einlassanschluss einströmen zu lassen. Die Patientenöffnung ist dazu eingerichtet, eine durchströmbare Verbindung zu einem Patienten bereitzustellen.

In einer Ausführungsform ist durch Verwendung weniger Bauteile, darunter eines Wegeventils, vorzugsweise eines 3/2-Wegeventils, zwischen einer fördernden und einer vibrierenden Fluidströmung unter Verwendung nur eines Kompressors umschaltbar.

In einer Ausführungsform ist ein Ventil vorgesehen, zwischen einem Kompressor und einem Vernebler angeordnet zu sein.
In einer Ausführungsform ist der Fluidanschluss in der Vibrations-Schaltstellung gesperrt. So kann eine Aerosolerzeugung in einer Vibrationsphase unterbunden werden.

Der Fluidanschluss ist gesperrt, wenn keine oder nahezu keine durchströmbare Verbindung des Fluidanschlusses mit dem Auslassanschluss vorhanden ist.

In einer Ausführungsform ist die Inhaliervorrichtungskomponente derart ausgebildet, dass die Schalteinrichtung mit einem Sensor verbindbar ist, der Sensor dazu eingerichtet ist, eine Atemphase zu erfassen und ein Atemsignal bereitzustellen und die Schalteinrichtung eingerichtet ist, das Atemsignal zu empfangen und das Vibrationsventil bei Empfang des Atemsignals in die Vibrations-Schaltstellung oder in die Aerosolerzeugungs-Schaltstellung zu schalten.

So kann die Umschaltung in die Vibrations-Schaltstellung auf besonders komfortable Weise erreicht werden.

Der Sensor weist vorzugsweise einen Drucksensor, einen Flusssensor, einen Feuchtigkeitssensor, einen Temperatursensor oder eine Kombination daraus auf.

Das Atemsignal weist vorzugsweise ein elektronisches, mechanisches, pneumatisches oder elektrisches Signal oder eine Kombination davon auf. Zweckmäßigerweise ist das Atemsignal dazu vorgesehen, in einer bestimmten Atemphase erzeugt zu werden. Vorzugsweise ist das Atemsignal ein Ausatmungssignal, das dazu vorgesehen ist, während einer Ausatmung erzeugt zu werden. In einer Ausführungsform ist das Atemsignal ein Einatmungssignal, das dazu vorgesehen ist, während einer Einatmung erzeugt zu werden.

In einer Ausführungsform ist die Schalteinrichtung dazu eingerichtet, das Vibrationsventil in die Aerosolerzeugungs-Schaltstellung zu schalten, wenn kein Atemsignal oder Ausatmungssignal vorliegt. Bevorzugt ist das Vibrationsventil mit einer Rückstellung, vorzugsweise einer Feder oder einem Magneten versehen, die das Vibrationsventil in die Aerosolerzeugungs-Schaltstellung schaltet, wenn die Schalteinrichtung das Vibrationsventil nicht in die Vibrationserzeugungs-Schaltstellung schaltet.

Zweckmäßigerweise ist der Sensor an einer Atemflussschnittstelle angeordnet, die dazu eingerichtet ist, von einem Atemfluss durchströmt zu werden. Die Atemflussschnittstelle umfasst in einer Ausführungsform einen Bereich einer Inhaliervorrichtung oder einer Inhaliervorrichtungskomponente, einen Schlauch, ein Nasenansatzstück, ein Mundstück oder eine Maske.

Es ist vorteilhaft, wenn die Atemflussschnittstelle einen Strömungswiderstand aufweist, der dazu geeignet ist, ein Gaumensegel zu schließen. In einer Ausführungsform ist die Höhe des Strömungswiderstandes einstellbar.

In einer besonders bevorzugten Ausführungsform ist in einem Ausatempfad ein leichter Widerstand, zweckmäßigerweise ein Strömungswiderstand vorhanden. Der Ausatempfad ist zweckmäßigerweise dazu vorgesehen, dass ein Anwender durch den Mund in den Ausatempfad ausatmet. Vorzugsweise ist der Sensor wirksam mit dem Ausatempfad verbunden. Zweckmäßigerweise ist ein leichter Widerstand vorgesehen, der dazu geeignet ist, dass bei einer Atmung dagegen das Gaumensegel geschlossen wird. Bei einem geschlossenen Gaumensegel kann eine erhöhte Deposition im Bereich der Nebenhöhlen erreicht werden. Durch den Strömungswiderstand kann eine zuverlässige und angenehme Möglichkeit bereitgestellt werden, das Gaumensegel zu schließen. Zweckmäßigerweise ist der Strömungswiderstand dazu ausgelegt, im Atemstrom einen Druck von 5 bis 20 cmWS oder etwa 500 bis 2000 Pa zu erzielen.

Es ist möglich, durch eine intelligente Aerosolerzeugung und einen intelligenten Aerosoltransport die Strömung darauf abzustimmen, ob das Gaumensegel offen oder geschlossen ist. Der Strömungswiderstand ist zweckmäßigerweise so groß, dass er geeignet ist, das Gaumensegel eines Anwenders zu veranlassen zu schließen. Die Schließung des Gaumensegels kann ausgenutzt werden und in Kombination mit einer intelligenten Aerosolerzeugung und Strömungsvariation zu einer Verbesserung der Deposition in den Nasennebenhöhlen führen.

In einer Ausführungsform ist der Sensor dazu eingerichtet, sowohl Ein- als auch Ausatemphasen zu ermitteln. Es ist zweckmäßig, wenn die Schalteinrichtung ermittelte Ein- und Ausatemphasen berücksichtigt. In einer Ausführungsform ist die Schalteinrichtung eingerichtet, eine Abstimmung eines Transports und einer Vibration mit einem Atemmuster und einer Aerosolerzeugung vorzunehmen. Besonders bevorzugt ist eine Steuerung derart eingerichtet, dass ermittelte Ein- und Ausatemphasen genutzt werden können, um eine Aerosolförderung in die Nase und eine aufgeprägte vibrierende Strömung oder einen Strömungsmustererzeuger für den Transport in die Nebenhöhlen zeitlich so zu steuern, dass eine Aerosolförderung oder ein Aerosoltransport hauptsächlich in der Einatemphase stattfindet.

In einer Ausführungsform ist der Sensor an einer Patientenschnittstelle angeordnet, die einen Patientenschnittstelleneinlass und einen Patientenschnittstellenauslass aufweist und derart mit der Inhaliervorrichtung verbindbar ist, dass ein durchströmbarer Pfad von der Inhaliervorrichtung durch den Patientenschnittstelleneinlass und den Patientenschnittstellenauslass vorhanden ist. So kann die Patientenschnittstelle mit verschiedenen Inhaliervorrichtungen eingesetzt werden. Das Vibrationsventil kann auf hygienische Weise von verschiedenen Anwendern genutzt werden.

Die Patientenschnittstelle weist vorzugsweise ein Mundstück oder eine Maske, besonders bevorzugt ein Nasenansatzstück auf.

Die Aufgabe wird auch gelöst durch eine Vibrationsinhaliervorrichtung mit einer Inhaliervorrichtungskomponente wie oben beschrieben, und einer Inhaliervorrichtung. Zweckmäßigerweise ist die Inhaliervorrichtung mit einer Vibrationsschnittstelle versehen, und der Vibrationseinlassanschluss ist mit der Vibrationsschnittstelle der Inhaliervorrichtung durchströmbar verbindbar.

Auf diese Weise kann eine kompakte Einheit aus Inhaliervorrichtung und Vibrationsventil bereitgestellt werden. Es ist vorteilhaft, wenn die Vibrationsinhaliervorrichtung derart kompakt ausgebildet ist, dass sie in einer Hand gehalten werden kann. In einer Ausführungsform ist die Vibrationsinhaliervorrichtung als Handheld-Gerät ausgebildet. Ein Handheld ist ein tragbares, elektronisches Gerät mit Stromversorgung über Akkus oder Batterien. Vorzugsweise ist die Einheit so klein und leicht, dass sie bei der Benutzung in nur einer Hand gehalten werden kann.

Bevorzugt ist ein Vibrationssystem mit einer Vibrationsinhaliervorrichtung wie oben beschrieben und einer Verdrängermaschine. Zweckmäßigerweise ist der Auslassanschluss mit einem Auslass der Verdrängermaschine durchströmbar verbindbar.

Auf diese Weise kann eine besonders kompakte Einheit aus Inhaliervorrichtung, Vibrationsventil und Verdrängermaschine bereitgestellt werden. In einer Ausführungsform ist das Vibrationssystem als Handheld-Gerät ausgebildet.

In einer vorteilhaften Ausführungsform ist die Verdrängermaschine ein Hubkolbenverdichter.

So kann auf besonders einfache Weise eine effektive Vibration in dem Vibrationssystem bereitgestellt werden. Ein Hubkolbenverdichter ist eine Maschine, die dazu vorgesehen ist, einem Gas mechanische Arbeit zuzuführen und den Druck und die Dichte des Gases zu erhöhen, indem ein Zylinder und ein hin- und herbewegbarer Kolben vorgesehen sind, so dass ein Gas von dem Kolben in den Arbeitsraum angesaugt, dort verdichtet und wieder ausgestoßen werden kann. Vorzugsweise sind Plattenventile als Ansaug- und Auslassventil vorgesehen.

Bevorzugt ist ein Vibrationssystem mit einer Inhaliervorrichtungskomponente wie oben beschrieben, einer Inhaliervorrichtung und einer Verdrängermaschine, wobei die Inhaliervorrichtung einen Fördereinlass aufweist, die Verdrängermaschine einen Auslass aufweist und eine durchströmbare Verbindung zwischen dem Fördereinlass und dem Auslass bereitstellbar ist. In einer Ausführungsform umfasst die durchströmbare Verbindung zwischen dem Fördereinlass und dem Auslass den Auslassanschluss, den Vibrationseinlassanschluss und die Verbindung zwischen dem Auslassanschluss und dem Vibrationseinlassanschluss.

In einer Ausführungsform ist die durchströmbare Verbindung zwischen dem Fördereinlass, dem Auslassanschluss und dem Auslass bereitstellbar.

So kann ein einfaches Vibrationsventil besonders effizient genutzt werden.

Es ist vorteilhaft, wenn die durchströmbare Verbindung zwischen dem Fördereinlass und dem Auslass einen höheren Strömungswiderstand aufweist als die durchströmbare Verbindung zwischen der Vibrationsschnittstelle und dem Auslass. So kann erreicht werden, dass in einem Zustand, in dem die Verbindung zwischen der Vibrationsschnittstelle und dem Auslass freigegeben ist, ein Fluid durch diese Verbindung strömt, und wenig oder gar kein Fluid die durchströmbare Verbindung zwischen dem Fördereinlass und dem Auslass durchströmt. So kann eine Fluidströmung zwischen Fördereinlass und Auslass verhindert oder begrenzt werden, ohne hier ein Ventil vorsehen zu müssen.

In einer Ausführungsform weist die durchströmbare Verbindung zwischen der Vibrationsschnittstelle und dem Auslass ein durchströmbares Bauteil mit einem durchströmbaren Querschnitt auf, wobei der durchströmbare Querschnitt durch Krafteinwirkung veränderbar ist. So kann eine Strömung zu der Vibrationsschnittstelle auf besonders einfache Weise unterbrochen werden. Vorzugsweise ist der durchströmbare Querschnitt durch Krafteinwirkung reduzierbar. Es ist vorteilhaft, wenn die Krafteinwirkung durch Körperkraft, Fingerkraft, einen Aktor, ein Getriebe, einen Hebelmechanismus, magnetische Energie, elektrische Energie, hydraulische Energie, pneumatische Energie, mechanische Energie oder eine Kombination daraus erzielbar ist. In einer besonders bevorzugten Ausführungsform ist das durchströmbare Bauteil ein Schlauch, der durch Fingerkraft zusammendrückbar ist. Zweckmäßigerweise weist der Schlauch einen elastischen Bereich auf.

In einer Ausführungsform ist eine durchströmbare Verbindung zwischen dem Fördereinlass und dem Fluidanschluss vorhanden. So kann eine Strömung zu dem Fördereinlass auf besonders einfache Weise durch Schalten des Vibrationsventils unterbrochen werden.

Die Aufgabe wird auch gelöst durch eine Verwendung der Inhaliervorrichtungskomponente, wobei der Auslassanschluss an einen Auslass einer Verdrängermaschine angeschlossen wird, der Vibrationseinlassanschluss an eine Vibrationsschnittstelle angeschlossen wird und die Schalteinrichtung zum Schalten des Vibrationsventils betätigt wird.

Es ist vorteilhaft, wenn die Schalteinrichtung betätigt wird, um zwischen einer Aerosolerzeugung und einer Bereitstellung einer Vibration zu wechseln.

In einer Ausführungsform wird die Schalteinrichtung betätigt, um zwischen einer Aerosolförderung und einer Bereitstellung einer Vibration zu wechseln.

In einer Ausführungsform wird die Schalteinrichtung betätigt, um zwischen Bereitstellen und Sperren einer Vibration zu wechseln.

In einer Ausführungsform weist die Vibrationsinhaliervorrichtung einen Taster auf, der dazu eingerichtet ist, das Ventil bei einer vorzugsweise manuellen Betätigung in die erste Schaltstellung zu schalten.

In Versuchen hat sich gezeigt, dass durch die Anwendung der Erfindung eine nachweisliche Menge an isotonischer Kochsalzlösung oder einer Lösung mit 9 g Kochsalz pro Liter Wasser in die Nasennebenhöhlen gelangen kann. Die Versuche bestätigen das Funktionieren der Erfindung.

Im Folgenden wird die Erfindung anhand von Ausführungsbeispielen unter Bezugnahme auf die beigefügten Figuren genauer beschrieben.
Figur 1 zeigt schematisch eine bevorzugte Ausführungsform der Inhaliervorrichtungskomponente,
Figur 2 zeigt ein Vibrationssystem mit der in Figur 1 gezeigten Inhaliervorrichtungskomponente in einer Aerosolerzeugungs-Schaltstellung,
Figur 3 zeigt ein Vibrationssystem mit der in Figur 1 gezeigten Inhaliervorrichtungskomponente in einer Vibrations-Schaltstellung,
Figur 4 zeigt schematisch eine bevorzugte Ausführungsform des Vibrationsventils,
Figur 5 zeigt ein Vibrationssystem mit der in Figur 4 gezeigten Inhaliervorrichtungskomponente 30 in der Aerosolerzeugungs-Schaltstellung.
Figur 6 zeigt ein Vibrationssystem mit der in Figur 4 gezeigten Inhaliervorrichtungskomponente in der Vibrations-Schaltstellung,
Figur 7 zeigt schematisch eine Ausführungsform der Inhaliervorrichtungskomponente,
Figur 8 zeigt ein Vibrationssystem mit dem in Figur 7 gezeigten Vibrationsventil in der Aerosolerzeugungs-Schaltstellung,
Figur 9 zeigt ein Vibrationssystem mit dem in Figur 7 gezeigten Vibrationsventil in der Vibrations-Schaltstellung,
Figur 10 zeigt schematisch eine bevorzugte Ausführungsform des Vibrationsystems,
Figur 11 zeigt schematisch ein Vibrationssystem mit einer Ventilbox,
Figur 12 zeigt schematisch ein Vibrationssystem mit einem an einem Kompressorgehäuse angebundenen Vibrationsventil,
Figur 13 zeigt schematisch ein Vibrationssystem mit einem in ein Kompressorgehäuse integriertes Vibrationsventil,
Figur 14 zeigt schematisch ein Vibrationssystem mit einem Membranvernebler, einem in eine Ventilbox integrierten Vibrationsventil und einer Verdrängermaschine,
Figur 15 zeigt schematisch ein weiteres Vibrationssystem mit einem Membranvernebler, einem in eine Ventilbox integrierten Vibrationsventil und einer Verdrängermaschine,
Figur 16 zeigt schematisch ein Vibrationssystem mit einer Inhalierhilfe, einem in eine Ventilbox integrierten Vibrationsventil und einer Verdrängermaschine,
Figur 17 beschreibt ein Ausführungsbeispiel eines Musters für Aerosolförderung und Vibration,
Figur 18 zeigt schematisch ein Ausführungsbeispiel eines an einem Kolbenkompressor vorgesehenen Vibrationsventils in einer Aerosolerzeugungs-Schaltstellung,
Figur 19 zeigt schematisch ein Ausführungsbeispiel eines an einem Kolbenkompressor vorgesehenen Vibrationsventils in einer Vibrationserzeugungs-Schaltstellung,
Figur 20 zeigt ein Vibrationssystem, das dem in Figur 11 gezeigten Vibrationssystem ähnelt.

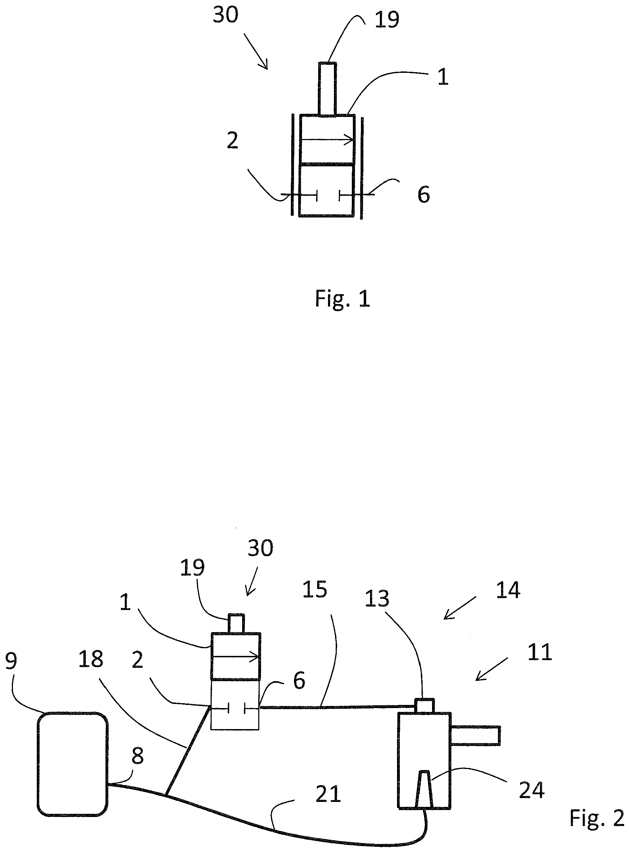

Figur 1 zeigt schematisch eine bevorzugte Ausführungsform der Inhaliervorrichtungskomponente 30. Die Inhaliervorrichtungskomponente 30 weist ein Vibrationsventil 1 mit einem Luftauslassanschluss 2, einem Vibrationsanschluss 6, einer Aerosolerzeugungs-Schaltstellung und einer Vibrations-Schaltstellung auf. In der Aerosolerzeugungs-Schaltstellung ist der Luftauslassanschluss 2 gesperrt. In der Vibrations-Schaltstellung ist der Luftauslassanschluss 2 mit dem Vibrationsanschluss 6 verbunden. An dem Vibrationsventil 1 ist ein Griff 19 vorgesehen.

Der Luftauslassanschluss 2 ist dazu vorgesehen, mit einem nicht gezeigten Auslass einer Verdrängermaschine verbunden zu werden. Der Vibrationsanschluss 6 ist dazu vorgesehen, mit einer nicht gezeigten Vibrationsschnittstelle eines Verneblers verbunden zu werden.

Das Vibrationsventil 1 ist dazu vorgesehen, in Verbindung mit einer nicht gezeigten Verdrängermaschine und einem nicht gezeigten Vernebler je nach Schaltzustand eine Aerosolerzeugung einzustellen, oder eine Vibration weiterzuleiten. Es ist dazu vorgesehen, bei einer Nutzung geschaltet zu werden, so dass diese Zustände abwechselnd geschaltet werden können. In diesem Ausführungsbeispiel ist das Vibrationsventil 1 dazu eingerichtet, manuell gesteuert zu werden. Das Vibrationsventil 1 kann auch dazu eingerichtet sein, elektrisch, pneumatisch, hydraulisch, mechanisch, magnetisch, atemzuggesteuert oder mit einer Kombination daraus geschaltet zu werden.

Figur 2 zeigt ein Vibrationssystem 14 mit der in Figur 1 gezeigten Inhaliervorrichtungskomponente 30 in der Aerosolerzeugungs-Schaltstellung. In der Aerosolerzeugungs-Schaltstellung verbindet das Vibrationsventil 1 die Anschlüsse folgendermaßen. Der Luftauslassanschluss 2 ist über einen Kompressorschlauch 18 mit einem Luftauslass 8 einer Verdrängermaschine 9 verbunden und der Vibrationsanschluss 6 ist über einen Vibrationsschlauch 15 mit einer Vibrationsschnittstelle 13 des Düsenverneblers 11 verbunden. Zwischen dem Luftauslass 8 des Kompressors 9 und einer Düse 24 des Düsenverneblers 11 ist ein durchströmbarer Schlauch 21 vorhanden. Der Schlauch 21 ist schaltventillos ausgebildet. Die durchströmbare Verbindung zu der Düse wird bei der vorgesehenen Anwendung des Vibrationssystems 14 nicht unterbrochen.

Im Betrieb des Vibrationssystems 14 gelangt Luft aus der Umgebung in die Verdrängermaschine 9. Verdichtete Druckluft wird zur Aerosolerzeugung durch den Luftauslass 8 und den Schlauch 21 direkt an die Düse 24 des Düsenverneblers 11 geleitet. Das Vibrationsventil 1 sperrt den Strömungsweg zur Vibrationsschnittstelle 13 des Düsenverneblers 11. Mit dem Griff 19 kann das Vibrationsventil 1 in die Vibrations-Schaltstellung geschaltet werden.

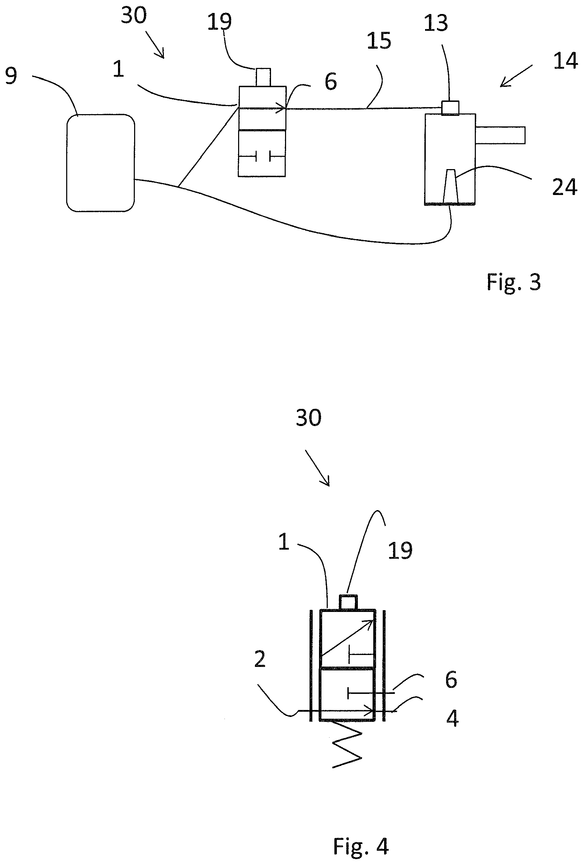

Figur 3 zeigt ein Vibrationssystem 14 mit der in Figur 1 gezeigten Inhaliervorrichtungskomponente 30 in einer Vibrations-Schaltstellung. Im Betrieb des Vibrationssystems 14 gelangt Luft aus der Umgebung in die Verdrängermaschine 9. Das Vibrationsventil 1 ist in Richtung der Vibrationsschnittstelle 13 offen. Da der Strömungswiderstand der Düse 24 größer ist als der Strömungswiderstand des Vibrationsanschlusses 6, strömt Luft durch den Vibrationsschlauch 15. In der Verdrängermaschine 9 findet keine signifikante Verdichtung statt. Aus diesem Grund wird die Luft in Übereinstimmung mit dem Hubzyklus ausgeschoben. Es entsteht eine Vibration. Mit dem Griff 19 kann das Vibrationsventil 1 in die Aerosolerzeugungs-Schaltstellung geschaltet werden.

Da in dem in den Figuren 2 und 3 gezeigten Vibrationssystem 14 eine schaltventillose Luftzuführung zu der Düse 24 vorhanden ist, wird bei laufender Verdrängermaschine 9 unabhängig von den Schaltzuständen des Vibrationsventils Aerosol erzeugt. In der Aerosolerzeugungs-Schaltstellung wird der Düse 24 mehr Luft zugeführt als in der Vibrations-Schaltstellung, da in der Vibrations-Schaltstellung ein Teil der Luft für die Vibrationen abgezweigt wird. Dieses Vibrationssystem 14 ist daher dazu eingerichtet, einer Aerosolerzeugung Vibrationen zu überlagern.

Es ist möglich, den Strömungswiderstand der Düse 24 so hoch vorzusehen, dass in der Vibrations-Schaltstellung keine Luft durch die Düse 24 strömt. Bei dieser Auswahl der Düse 24 ist das Vibrationssystem 14 dazu eingerichtet, abwechselnd Aerosolerzeugung und Vibration vorzusehen.

Figur 4 zeigt schematisch eine bevorzugte Ausführungsform des Vibrationsventils 1. Das Vibrationsventil 1 weist einen Luftauslassanschluss 2, einen Fluidanschluss 4, einen Vibrationsanschluss 6, eine Aerosolerzeugungs-Schaltstellung und eine Vibrations-Schaltstellung auf.

In der Aerosolerzeugungs-Schaltstellung ist der Luftauslassanschluss 2 mit dem Fluidanschluss 4 verbunden. Der Vibrationsanschluss 6 ist gesperrt. In der Vibrations-Schaltstellung ist der Luftauslassanschluss 2 mit dem Vibrationsanschluss 6 verbunden. Der Fluidanschluss 4 ist gesperrt. Zur Betätigung des Vibrationsventils 1 ist ein Taster 19 vorgesehen.

Der Luftauslassanschluss 2 ist dazu vorgesehen, mit einem nicht gezeigten Auslass eines Kompressors verbunden zu werden. Der Fluidanschluss 4 ist dazu vorgesehen, mit einer nicht gezeigten Düse eines Düsenverneblers verbunden zu werden. Der Vibrationsanschluss 6 ist dazu vorgesehen, mit einer nicht gezeigten Vibrationsschnittstelle eines Verneblers verbunden zu werden.

Das Vibrationsventil 1 ist dazu vorgesehen, in Verbindung mit einer Verdrängermaschine und einem Düsenvernebler je nach Schaltzustand eine Aerosolerzeugung einzustellen oder eine Vibration zu leiten. Es ist dazu vorgesehen, bei einer Nutzung geschaltet zu werden, so dass diese Zustände abwechselnd eingestellt werden können. In diesem Ausführungsbeispiel ist das Vibrationsventil 1 dazu eingerichtet, manuell gesteuert zu werden. Das Vibrationsventil 1 kann auch dazu eingerichtet sein, elektrisch, mechanisch, pneumatisch, hydraulisch, magnetisch, atemzuggesteuert oder mit einer Kombination daraus geschaltet zu werden.

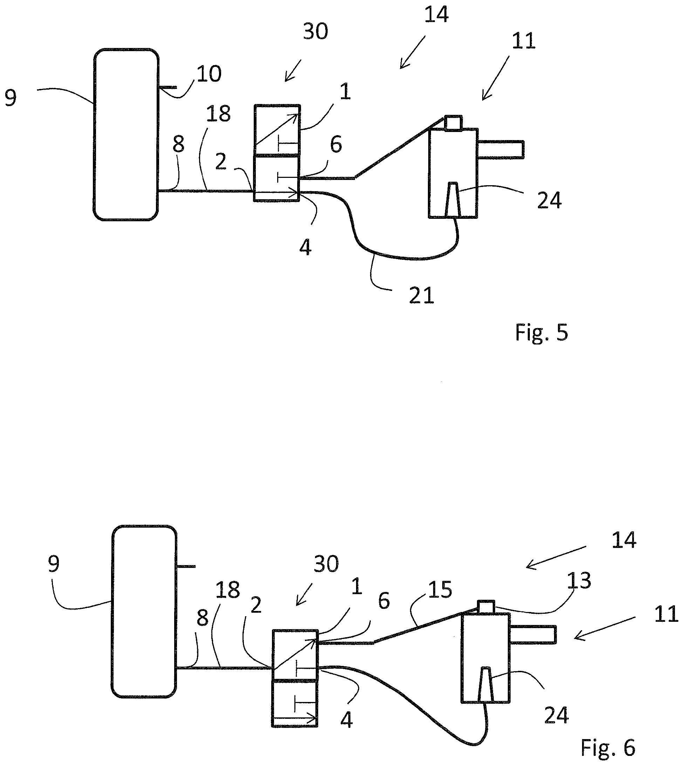

Figur 5 zeigt ein Vibrationssystem 14 mit der in Figur 4 gezeigten Inhaliervorrichtungskomponente 30 in der Aerosolerzeugungs-Schaltstellung. Das Vibrationsventil 1 ist zwischen einem Kompressor 9 und einem Düsenvernebler 11 angeordnet. Der Luftauslassanschluss 2 ist über einen Kompressorschlauch 18 mit einem Luftauslass 8 eines Kompressors 9 verbunden, der Fluidanschluss 4 ist über einen Schlauch 21 mit einer Düse 24 eines Düsenverneblers 11 verbunden und der Vibrationsanschluss 6 ist gesperrt. Der Lufteinlass 10 des Kompressors 9 ist nicht mit dem Vibrationsventil 1 verbunden.

Im Betrieb des Vibrationssystems 14 gelangt Luft aus der Umgebung in den Kompressor 9. Die Luft wird in dem Kompressor 9 komprimiert und verdichtete Druckluft wird durch den Luftauslass 8 und den Kompressorschlauch 18 in das Vibrationsventil 1 geleitet. Hierüber erreicht die Druckluft die Düse 24 des Düsenverneblers 11 zur Aerosolerzeugung.

Figur 6 zeigt ein Vibrationssystem 14 mit der in Figur 4 gezeigten Inhaliervorrichtungskomponente 30 in der Vibrations-Schaltstellung. Der Luftauslassanschluss 2 ist über den Kompressorschlauch 18 mit einem Luftauslass 8 eines Kompressors 9 verbunden, der Fluidanschluss 4 ist gesperrt und der Vibrationsanschluss 6 ist über einen ersten Vibrationsschlauch 15 mit einer Vibrationsschnittstelle 13 des Düsenverneblers 11 verbunden.

Im Betrieb des Vibrationssystems 14 gelangt Luft aus der Umgebung in den Kompressor 9. Die Luft wird ohne Verdichtungsprozess zum Vibrationsventil 1 geleitet. Mithilfe des Vibrationsventils 1 gelangt die Luft zur Vibrationsschnittstelle 13 des Düsenverneblers 11. Da kein Widerstand vergleichbar mit der Düse 24 des Düsenverneblers 11 wirkt, findet im Kompressor 9 keine signifikante Verdichtung statt. Aus diesem Grund wird die Luft entsprechend dem Hubzyklus ausgeschoben. Es entsteht eine Vibration.

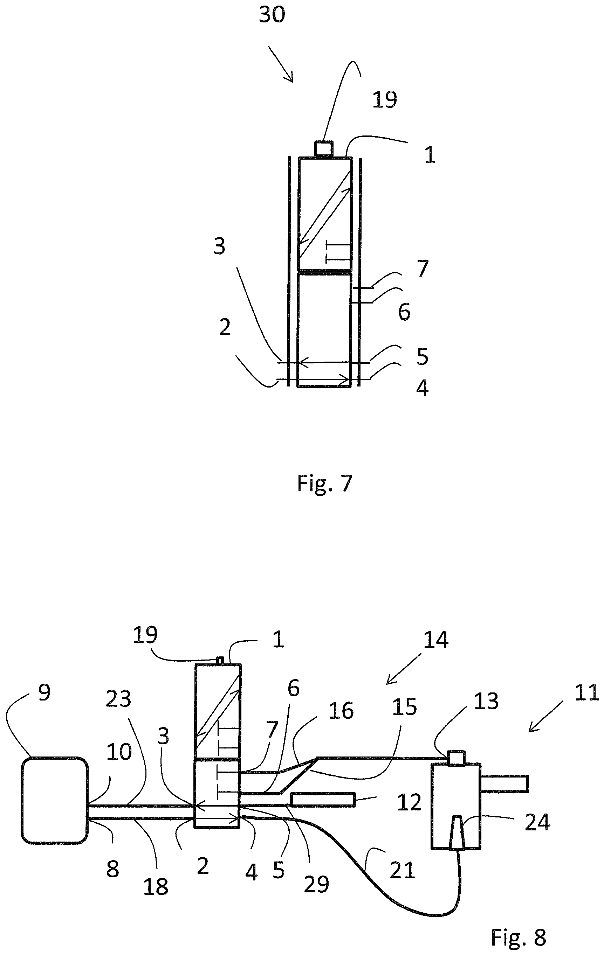

Figur 7 zeigt schematisch eine Ausführungsform der Inhaliervorrichtungskomponente 30. Die Inhaliervorrichtungskomponente 30 weist ein Vibrationsventil 1 mit einem Luftauslassanschluss 2, einem Lufteinlassanschluss 3, einem Fluidanschluss 4, einem Luftfilteranschluss 5, einem ersten Vibrationsanschluss 6 und einem zweiten Vibrationsanschluss 7 auf. Das Vibrationsventil 1 weist eine Aerosolerzeugungs-Schaltstellung und eine Vibrations-Schaltstellung auf.

Das Vibrationsventil 1 ist dazu vorgesehen, Anschlüsse zu verbinden. Dabei sind je nach Schaltzustand unterschiedliche Verbindungen vorgesehen. In der Aerosolerzeugungs-Schaltstellung ist der Luftfilteranschluss 5 mit dem Lufteinlassanschluss 3 durchströmbar verbunden. Der Luftauslassanschluss 2 ist mit dem Fluidanschluss 4 verbunden. In der Vibrations-Schaltstellung sind Fluidanschluss 4 und Luftfilteranschluss 5 gesperrt. Der erste Vibrationsanschluss 6 ist mit dem Luftauslassanschluss 2 verbunden. Der zweite Vibrationsanschluss 7 ist mit dem Lufteinlassanschluss 3 verbunden.

Die Anschlüsse sind dazu vorgesehen, mit einem Einlass und einem Auslass eines nicht gezeigten Kolbenkompressors, einem nicht gezeigten Luftfilter, einer nicht gezeigten Düse eines Düsenverneblers und einer nicht gezeigten Vibrationsschnittstelle eines Verneblers verbunden zu werden.

Figur 8 zeigt ein Vibrationssystem 14 mit dem in Figur 7 gezeigten Vibrationsventil 1 in der Aerosolerzeugungs-Schaltstellung. Der Luftauslassanschluss 2 ist mit dem Luftauslass 8 eines Kolbenkompressors 9 verbunden. Der Lufteinlassanschluss 3 ist mit einem Lufteinlass 10 des Kolbenkompressors 9 verbunden. Der Fluidanschluss 4 ist mit einer Düse 24 eines Düsenverneblers 11 verbunden. Der Luftfilteranschluss 5 ist mit einem Luftfilter 12 verbunden. Der erste Vibrationsanschluss 6 ist mit einer Vibrationsschnittstelle 13 des Düsenverneblers 11 verbunden. Der zweite Vibrationsanschluss 7 ist ebenfalls mit der Vibrationsschnittstelle 13 des Düsenverneblers 11 verbunden.

Die Verbindung von Luftauslassanschluss 2 und Luftauslass 8 ist mit einem Kompressorschlauch 18 hergestellt. Die Verbindung des Fluidanschlusses 4 mit der Düse 24 ist mit einem Schlauch 21 hergestellt. Die Verbindungen der Vibrationsanschlüsse 6, 7 mit der Vibrationsschnittstelle 13 sind mit einem ersten Vibrationsschlauch 15 und einem zweiten Vibrationsschlauch 16 hergestellt. Die Verbindung des Lufteinlassanschlusses 3 mit dem Lufteinlass 10 ist mit einem Lufteinlassschlauch 23 hergestellt. Die Verbindung des Luftfilteranschlusses 5 mit dem Luftfilter 12 ist über einen Luftfilterschlauch 29 hergestellt.

An dem Vibrationsventil 1 ist eine Schalteinrichtung 19 zum Schalten des Vibrationsventils 1 vorgesehen.

Im Betrieb des Vibrationssystems 14 wird Luft durch den Luftfilter 12, den Luftfilterschlauch 29, das Vibrationsventil 1 und den Lufteinlassschlauch 23 hindurch zu dem Lufteinlass 10 des Kolbenkompressors 9 gesaugt oder geleitet. Im Kolbenkompressor 9 verdichtete Druckluft wird über den Luftauslass 8 und den Kompressorschlauch 18 zu dem Vibrationsventil 1 geleitet und gelangt über den Schlauch 21 zur Aerosolerzeugung an die Düse 24 des Düsenverneblers 11.

Mit der Schalteinrichtung 19 kann das Vibrationsventil 1 in die Vibrations-Schaltstellung geschaltet werden.

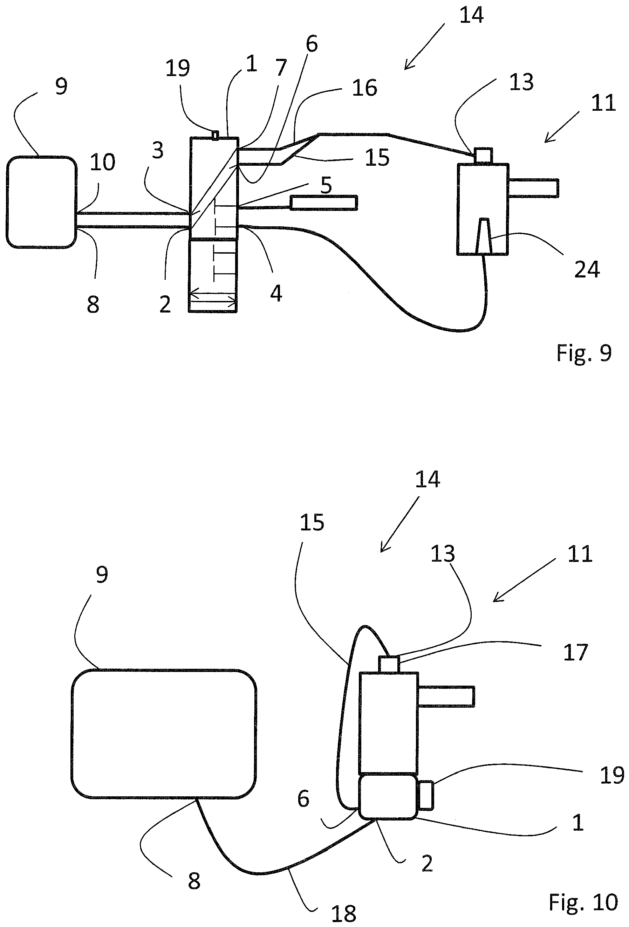

Figur 9 zeigt ein Vibrationssystem mit dem in Figur 7 gezeigten Vibrationsventil 1 in der Vibrations-Schaltstellung. Der Luftauslassanschluss 2 ist mit dem ersten Vibrationsanschluss 6 verbunden und der Lufteinlassanschluss 3 ist mit dem zweiten Vibrationsanschluss 7 verbunden. Der Luftfilteranschluss 5 ist gesperrt. Der Fluidanschluss 4 ist ebenfalls gesperrt.

In diesem Schaltzustand sind Lufteinlass 10 und Luftauslass 8 des Kolbenkompressors 9 mithilfe des Vibrationsventils 1, des ersten Vibrationsschlauchs 15, des zweiten Vibrationsschlauchs 16 und der Vibrationsschnittstelle 13 miteinander verbunden. Da an den Vibrationsanschlüssen 6, 7 oder der Vibrationsschnittstelle 13 kein Widerstand wirkt, der mit einem Widerstand an einer Düse 24 eines Düsenverneblers 11 vergleichbar ist, findet im Kolbenkompressor 9 keine signifikante Verdichtung statt. Bei einem Betrieb des Kolbenkompressors 9 stellt sich infolge des Kolbenhubs des Kolbenkompressors 9 eine Vibration ein, welche über die Vibrationsanschlüsse in den Düsenvernebler 11 eingeleitet wird. Luft wird von dem Kolben-Zylinder-System abwechselnd angesaugt und ausgeschoben, so dass eine Vibration entsteht.

Da der Fluidanschluss 4 gesperrt ist, wird in diesem Schaltzustand kein Aerosol erzeugt. Das Vibrationssystem 14 ist daher geeignet, abwechselnd Aerosol und Vibrationen zu erzeugen.

Figur 10 zeigt schematisch eine bevorzugte Ausführungsform des Vibrationsystems 14. Das Vibrationsventil 1 ist dazu eingerichtet, auf einen Vernebler 11 aufgesteckt zu werden, so dass eine Einheit aus Vibrationsventil 1 und Vernebler 11 vorhanden ist, welche in einer Hand gehalten werden kann. An dem Vibrationsventil 1 ist ein Taster 19 angeordnet. Dieser Taster 19 ist dazu eingerichtet, bei einer Betätigung das Vibrationsventil 1 zu schalten. In der in Figur 10 gezeigten Ausführungsform ist der Taster 19 dazu vorgesehen, bei einer Betätigung in eine Vibrations-Schaltstellung zu schalten. Das Vibrationsventil 1 ist dazu eingerichtet die Aerosolerzeugungs-Schaltstellung einzunehmen, wenn der Taster 19 nicht gedrückt wird. Das Vibrationsventil 1 weist einen Luftauslassanschluss 2 auf, der dazu vorgesehen ist, über einen Kompressorschlauch 18 mit einem Auslass 8 einer Verdrängermaschine 9 verbunden zu werden. Das Vibrationsventil 1 weist einen Vibrationsanschluss 6 auf, der dazu eingerichtet ist, über einen Vibrationsschlauch 15 mit einer in dem Vernebler vorgesehenen Vibrationsschnittstelle 13 verbunden zu werden.

Zur Nutzung des Vibrationssystems 14 steckt ein Anwender den Vernebler 11 auf das Vibrationsventil 1 auf. Der Anwender verbindet einen Vibrationsschlauch 15 mit dem Vibrationsventil 1 und einem Vernebleroberteil 17. Der Anwender verbindet mit einem Kompressorschlauch 18 das Vibrationsventil 1 mit einer Verdrängermaschine 9. In der hier gezeigten Ausführungsform liegen die Schläuche 15 und 18 lose vor. Sie können auch fest integriert ausgeführt sein. Während der Anwendung kann der Anwender mit einem Taster 19 zwischen der Aerosolerzeugung und der Vibrationserzeugung wechseln.

Dem Vibrationssystem 14 liegt eine Bedienungsanleitung bei, in der der Anwender dazu angeleitet wird, beim Ausatmen durch den Mund die Vibration einzuschalten. Da beim Ausatmen durch den Mund das Gaumensegel geschlossen ist, ist dies ein günstiger Zeitraum für eine Nasennebenhöhlentherapie. Außerdem hat der Anwender eine gute Möglichkeit, die Bedienung des Schalters zu koordinieren. In anderen Ausführungsformen wird der Anwender auf andere Weise dazu angeleitet, beim Ausatmen durch den Mund die Vibration einzuschalten.

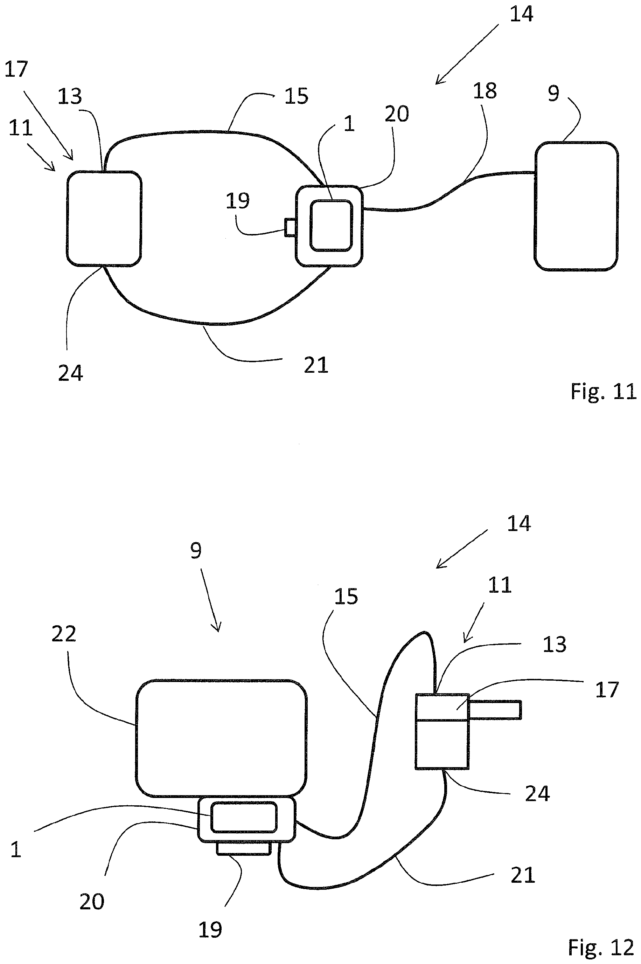

Figur 11 zeigt schematisch ein Vibrationssystem 14 mit einer Ventilbox 20. Das Vibrationssystem 14 ähnelt dem in Figur 10 gezeigten Vibrationssystem 14. Im Unterschied zu dem in Figur 10 gezeigten Vibrationssystem 14 ist das Vibrationsventil 1 nicht an dem Vernebler 11 befestigbar. Das Vibrationsventil 1 ist in die Ventilbox 20 eingebaut, die dazu vorgesehen ist, über Schläuche 15, 18, 21 mit einer Verdrängermaschine 9 und einem Düsenvernebler 11 verbunden zu werden. Bei Verwendung des Vibrationssystems 14 verbindet ein Anwender die Ventilbox 20 mit einem Kompressorschlauch 18 mit der Verdrängermaschine 9. Ein Vibrationsschlauch 15 wird mit der Ventilbox 20 und der Vibrationsschnittstelle 13 verbunden. Ein Druckschlauch 21 wird mit der Ventilbox 20 und einer Aerosolförderungsschnittstelle 24 des Verneblers 11 verbunden. Die Ventilbox 20 weist einen Schalter 19 auf, der dazu eingerichtet ist, das Vibrationsventil 1 bei einer Betätigung in eine andere Schaltstellung zu schalten.

Figur 12 zeigt schematisch ein Vibrationssystem 14 mit einem an einem Kompressorgehäuse 22 angebundenen Vibrationsventil 1. Das Vibrationsventil 1 ist als Ventilbox 20 direkt mit dem Kompressorgehäuse 22 verbunden.

Zur Verwendung verbindet der Anwender die Ventilbox 20 mit dem Kompressor 9. Es ist auch möglich, die Ventilbox 20 bereits in der Produktion mit dem Kompressor 9 zu verbinden.

Ein Vibrationsschlauch 15 ist mit der Ventilbox 20 und einer Vibrationsschnittstelle 13 in einem Vernebleroberteil 17 verbunden. Ein Schlauch 21 ist mit der Ventilbox 20 und einer Aerosolförderungsschnittstelle 24 eines Verneblers 11 verbunden.

In der in Figur 12 gezeigten Ausführungsform liegen die Schläuche 15, 21 lose vor. Die Schläuche 15, 21 können auch fest integriert in ein oder mehrere Bestandteile des Vibrationssystems 14 vorliegen. Es ist ein Taster 19 vorgesehen, mit dem ein Anwender während einer Therapie zwischen einer Aerosolerzeugung und der Vibrationserzeugung wechseln kann.

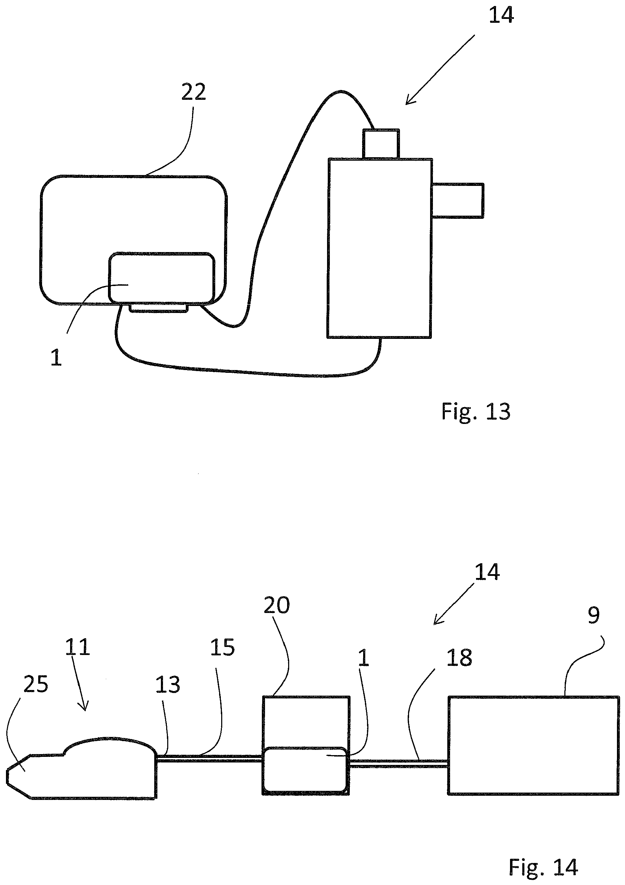

Figur 13 zeigt schematisch ein Vibrationssystem 14 mit einem in ein Kompressorgehäuse integrierten Vibrationsventil 1. Aufbau und Verwendung entsprechen dem in Figur 12 gezeigten Vibrationssystem 14.

Figur 14 zeigt schematisch ein Vibrationssystem 14 mit einem Membranvernebler 11, einem in eine Ventilbox 20 integrierten Vibrationsventil 1 und einer Verdrängermaschine 9. Die Ventilbox 20 ist über einen Kompressorschlauch 18 mit der Verdrängermaschine 9 verbunden. Ein Vibrationsschlauch 15 ist mit der Ventilbox 20 und einer Vibrationsschnittstelle 13 des Membranverneblers 11 verbunden. Die Vibrationsschnittstelle 13 weist einen geringen Strömungswiderstand auf, so dass von der Verdrängermaschine 9 erzeugte Vibrationen in den Membranvernebler 11 hinein und aus dem Nasenansatzstück 25 heraus geleitet werden können.

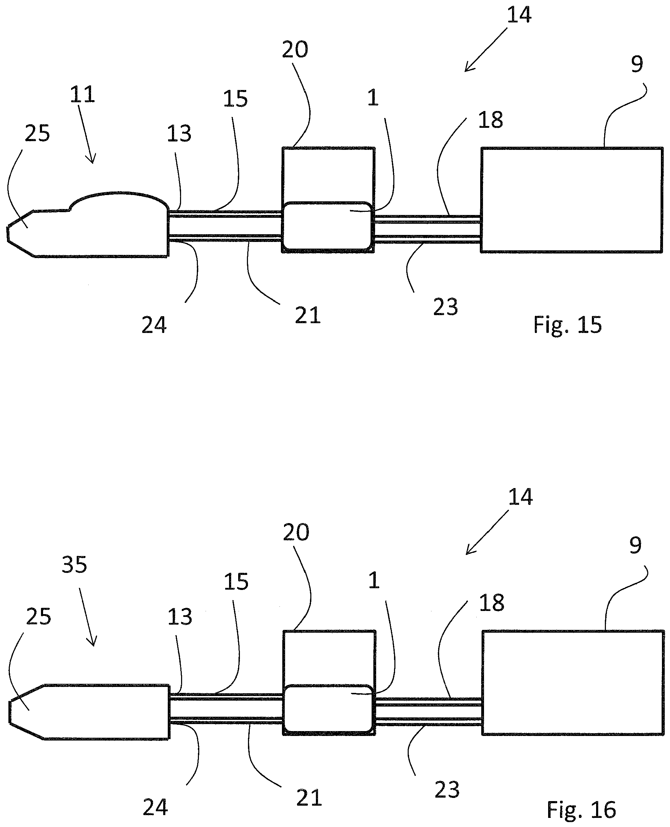

Figur 15 zeigt schematisch ein weiteres Vibrationssystem 14 mit einem Membranvernebler 11, einem in eine Ventilbox 20 integrierten Vibrationsventil 1 und einer Verdrängermaschine 9. Das in Figur 15 gezeigte Vibrationssystem 14 ähnelt im Aufbau dem in Figur 14 gezeigten Vibrationssystem 14. Im Unterschied zu dem in Figur 14 gezeigten Vibrationssystem 14 weist das in Figur 15 gezeigte Vibrationssystem 14 einen Lufteinlassschlauch 23 und einen Schlauch 21 auf. Der Lufteinlassschlauch 23 verbindet die Verdrängermaschine 9 mit dem Vibrationsventil 1. Der Schlauch 21 verbindet eine Aerosolförderungsschnittstelle 24 mit dem Vibrationsventil 1.

Die Verdrängermaschine 9 kann Fluid ein- und ausschieben. Als Verdrängermaschine 9 kann ein Kompressor eingesetzt sein. Dadurch kann Fluid in den Membranvernebler 11 hinein und aus dem Membranvernebler 11 heraus gefördert werden. Es kann eine reine Vibration ohne Förderung erzielt werden. Ein Anwender kann zwischen reiner Vibration und vibrierender Förderung umschalten.

Figur 16 zeigt schematisch ein Vibrationssystem 14 mit einer Inhalierhilfe 35, einem in eine Ventilbox 20 integrierten Vibrationsventil 1 und einer Verdrängermaschine 9. Aufbau und Funktion ähneln dem in Figur 15 gezeigten Vibrationssystem 14. Im Unterschied zu dem in Figur 15 gezeigten Vibrationssystem 14 ist hier statt des Membranverneblers 11 eine Inhalierhilfe 35 eingesetzt.

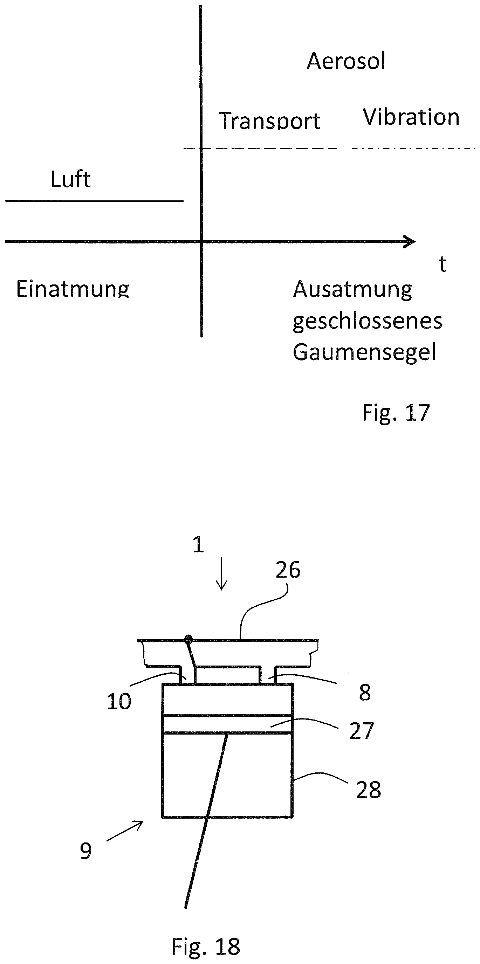

Figur 17 beschreibt ein Ausführungsbeispiel eines Musters für Aerosolförderung und Vibration. Während der Einatmung wird dem Anwender Luft zugeführt. Während der Ausatmung wird das Gaumensegel geschlossen. Dies kann willentlich oder durch Ausatmen durch den Mund gegen einen Widerstand erfolgen. Während der Ausatemphase wird Aerosol zunächst transportiert und dann vibriert.

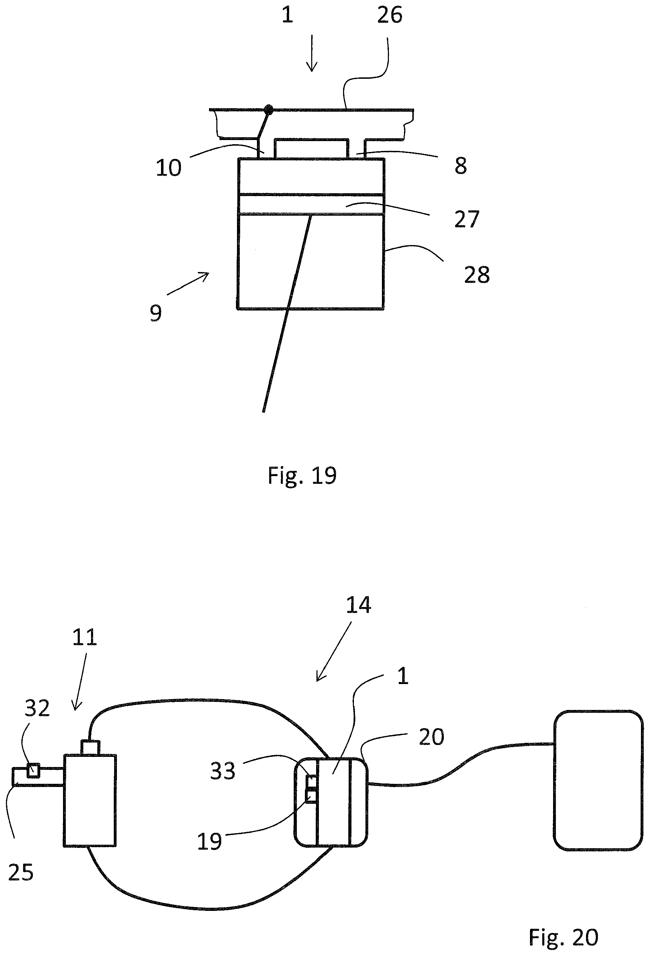

Die Figuren 18 und 19 zeigen schematisch ein Ausführungsbeispiel eines an einem Kolbenkompressor 9 vorgesehenen Vibrationsventils 1. Figur 18 zeigt das Vibrationsventil in einer Aerosolerzeugungs-Schaltstellung und Figur 19 zeigt das Vibrationsventil in einer Vibrationserzeugungs-Schaltstellung. Das Vibrationsventil 1 ist derart eingerichtet, dass es in einer Vibrations-Schaltstellung eine Verbindung eines Einlass 10 des Kolbenkompressors 9 mit einer Umgebung sperrt und eine Verbindungsleitung 26 zwischen Einlass 10 und Auslass 8 freigibt und in einer Aerosolerzeugungs-Schaltstellung die Verbindung zwischen dem Einlass 10 und der Umgebung freigibt und die Verbindungsleitung 26 sperrt.

In der Vibrations-Schaltstellung kann der Kolben 27 durch den Einlass 10 aus der Umgebung kein Gas ansaugen. Das sich im Kolbenraum 28 und in der Verbindungleitung 26 befindende Gas wird mit dem Kolbenhub hin- und hergeschoben. In der Aerosolerzeugungs-Schaltstellung zieht der Kolben 27 durch den Einlass 10 aus der Umgebung Gas ein, komprimiert es und schiebt es durch den Auslass 8 aus dem Kolbenraum 28 heraus.

Figur 20 zeigt ein Vibrationssystem 14, das dem in Figur 11 gezeigten Vibrationssystem 14 ähnelt. Im Unterschied zu dem in Figur 11 gezeigten Vibrationssystem 14 weist der Vernebler 11 ein Nasenansatzstück 25 auf, das mit einem Strömungssensor 32 versehen ist. Der Strömungssensor 32 ist dazu eingerichtet, eine Ausatmung anhand der Strömungsrichtung zu detektieren und ein Ausatmungssignal an einen in der Ventilbox 20 vorgesehenen Empfänger 33 zu senden. Der Empfänger 33 ist dazu eingerichtet, bei vorhandenem Ausatmungssignal einen Aktuator 19 zu aktivieren, der das Vibrationsventil 1 in eine Vibrations-Schaltstellung schaltet. Das Vibrationsventil weist einen Rückstellmechanismus auf, der dazu eingerichtet ist, das Vibrationsventil 1 aus der Vibrations-Schaltstellung in die Aerosolerzeugungs-Schaltstellung zu schalten, wenn der Aktuator inaktiv ist.

## Patentansprüche

1. Inhaliervorrichtungskomponente (30) mit
einem Auslassanschluss (2), der dazu eingerichtet ist, mit einem Auslass (8) einer Verdrängermaschine (9) durchströmbar verbunden zu werden, und
einem Vibrationseinlassanschluss (6), der dazu eingerichtet ist, mit einer Vibrationsschnittstelle (13) durchströmbar verbunden zu werden,
einem Vibrationsventil (1), das
eine Vibrations-Schaltstellung, in der der Auslassanschluss (2) mit dem Vibrationseinlassanschluss (6) durchströmbar verbunden ist, und
eine Aerosolerzeugungs-Schaltstellung, in der die Verbindung zwischen Auslassanschluss (2) und Vibrationseinlassanschluss (6) gesperrt ist, aufweist, und einer Schalteinrichtung (19) zum Schalten des Vibrationsventils (1).

2. Inhaliervorrichtungskomponente (30), nach Anspruch 1, **dadurch gekennzeichnet, dass** das Vibrationsventil (1) einen Fluidanschluss (4) aufweist, der dazu eingerichtet ist, mit einem Fördereinlass (24) einer Inhaliervorrichtung (11) durchströmbar verbunden zu werden, und in der Aerosolerzeugungs-Schaltstellung der Auslassanschluss (2) mit dem Fluidanschluss (4) durchströmbar verbunden ist.

3. Inhaliervorrichtungskomponente (30) nach Anspruch 1 oder Anspruch 2, **dadurch gekennzeichnet, dass** das Vibrationsventil (1) einen durchströmbaren Einlassanschluss (3) aufweist, der dazu eingerichtet ist, mit einem Einlass (10) der Verdrängermaschine (9) durchströmbar verbunden zu werden, und
in der Vibrations-Schaltstellung der Einlassanschluss (3), der Auslassanschluss (2) oder beide mit der Vibrationsschnittstelle (13) durchströmbar verbindbar sind.

4. Inhaliervorrichtungskomponente (30) nach Anspruch 2 oder Anspruch 3, **dadurch gekennzeichnet, dass** die Verbindung zwischen Auslassanschluss (2) und Fluidanschluss (4) in der Vibrations-Schaltstellung gesperrt ist.

5. Inhaliervorrichtungskomponente (30) nach einem der vorhergehenden Ansprüche, **dadurch gekennzeichnet, dass** die Schalteinrichtung (19) mit einem Sensor (32) verbindbar ist,
der Sensor (32) dazu eingerichtet ist, eine Atemphase zu erfassen und ein Atemsignal bereitzustellen und
die Schalteinrichtung (19) eingerichtet ist, das Atemsignal zu empfangen und das Vibrationsventil (1) bei Empfang des Atemsignals in die Vibrations-Schaltstellung oder in die Aerosolerzeugungs-Schaltstellung zu schalten.

6. Inhaliervorrichtungskomponente (30) nach einem der vorhergehenden Ansprüche, **dadurch gekennzeichnet, dass** der Sensor (32) an einer Atemflussschnittstelle angeordnet ist, die dazu eingerichtet ist, von einem Atemfluss durchströmt zu werden.

7. Inhaliervorrichtungskomponente (30) nach Anspruch 6, **dadurch gekennzeichnet, dass** die Atemflussschnittstelle einen Strömungswiderstand aufweist, der dazu geeignet ist, ein Gaumensegel zu schließen.

8. Inhaliervorrichtungskomponente (30) nach einem der Ansprüche 5 bis 7, **dadurch gekennzeichnet, dass** der Sensor (32) an einer Patientenschnittstelle (25) angeordnet ist, die einen Patientenschnittstelleneinlass und einen Patientenschnittstellenauslass aufweist und derart mit der Inhaliervorrichtung (11) verbindbar ist, dass ein durchströmbarer Pfad von der Inhaliervorrichtung (11) durch den Patientenschnittstelleneinlass und den Patientenschnittstellenauslass vorhanden ist.

9. Vibrationsinhaliervorrichtung mit einer Inhaliervorrichtungskomponente (30) nach einem der Ansprüche 1 bis 8 und einer Inhaliervorrichtung (11).

10. Vibrationssystem (14) mit einer Vibrationsinhaliervorrichtung nach Anspruch 9 und einer Verdrängermaschine (9).

11. Vibrationssystem (14) nach Anspruch 10, **dadurch gekennzeichnet, dass** die Inhaliervorrichtung (11) einen Fördereinlass (24) aufweist, die Verdrängermaschine (9) einen Auslass (8) aufweist und eine durchströmbare Verbindung zwischen dem Fördereinlass (24) und dem Auslass (8) bereitstellbar ist.

12. Vibrationssystem (14) nach Anspruch 10 oder Anspruch 11, **dadurch gekennzeichnet, dass** eine durchströmbare Verbindung zwischen dem Fördereinlass (24), dem Auslassanschluss (2) und dem Auslass (8) bereitstellbar ist.

13. Vibrationssystem (14) nach einem der Ansprüche 11 oder 12, **dadurch gekennzeichnet, dass** die durchströmbare Verbindung zwischen dem Fördereinlass (24) und dem Auslass (8) einen höheren Strömungswiderstand aufweist als die durchströmbare Verbindung zwischen der Vibrationsschnittstelle (13) und dem Auslass (8).

14. Vibrationssystem (14) nach einem der Ansprüche 11 bis 13, **dadurch gekennzeichnet, dass** die durchströmbare Verbindung zwischen der Vibrationsschnittstelle (13) und dem Auslass (8) ein durchströmbares Bauteil mit einem durchströmbaren Querschnitt aufweist, und der durchströmbare Querschnitt durch Krafteinwirkung veränderbar ist.

15. Verwendung einer Inhaliervorrichtungskomponente (30) nach einem der Ansprüche 1 bis 8,
wobei der Auslassanschluss (2) an einen Auslass (8) einer Verdrängermaschine (9) angeschlossen wird,
der Vibrationseinlassanschluss (6) an eine Vibrationsschnittstelle (13) angeschlossen wird und die Schalteinrichtung (19) zum Schalten des Vibrationsventils (1) betätigt wird.
